(19) Europäisches Patentamt
European Patent Office
Office européen des brevets

(11) EP 3 743 415 B1

(12) **FASCICULE DE BREVET EUROPEEN**

(45) Date de publication et mention de la délivrance du brevet:
**16.02.2022 Bulletin 2022/07**

(21) Numéro de dépôt: **19700955.8**

(22) Date de dépôt: **23.01.2019**

(51) Classification Internationale des Brevets (IPC):
***C07D 327/04*** $^{(2006.01)}$ ***C11B 9/00*** $^{(2006.01)}$

(52) Classification Coopérative des Brevets (CPC):
**C07D 327/04; C11B 9/0088**

(86) Numéro de dépôt international:
**PCT/EP2019/051597**

(87) Numéro de publication internationale:
**WO 2019/145347 (01.08.2019 Gazette 2019/31)**

(54) **NOUVEAUX COMPOSÉS SPIROOXATHIOLANONES, LEUR PROCÉDÉ DE PRÉPARATION AINSI QUE LEUR UTILISATION EN PARFUMERIE ET AROMATIQUE**

NEUE SPIROOXATHIOLANON-VERBINDUNGEN, VERFAHREN ZU IHRER HERSTELLUNG UND IHRE VERWENDUNG IN DER PARFÜM- UND AROMASTOFFBRANCHE

NEW SPIROOXATHIOLANONE COMPOUNDS, PROCESS FOR PREPARATION THEREOF, AND USE THEREOF IN THE PERFUMERY AND AROMATICS INDUSTRY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA MD**

(30) Priorité: **25.01.2018 FR 1850613**

(43) Date de publication de la demande:
**02.12.2020 Bulletin 2020/49**

(73) Titulaire: **V. Mane Fils**
**06620 Bar sur Loup (FR)**

(72) Inventeurs:
- **MURATORE, Agnès**
**06740 CHATEAUNEUF DE GRASSE (FR)**
- **GRASSET, Fabien**
**06130 GRASSE (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**EP-A1- 0 105 157**
**WO-A1-2005/042680**
**JP-A- 2001 039 972**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet de nouveaux composés de type spirooxathiolanones possédant des notes fruitées, pêche et/ou fruits exotiques, leur procédé de préparation, ainsi que leurs utilisations dans l'industrie de la chimie, et en particulier en parfumerie, cosmétique, parapharmacie, dans l'industrie des détergents, ainsi que dans l'alimentaire, lesdits composés présentant des propriétés organoleptiques intéressantes ainsi qu'une puissance et une rémanence particulières.

Afin d'accroître la gamme des notes disponibles des parfumeurs et aromaticiens pour accompagner leurs créations, l'industrie des parfums et arômes est en perpétuelle recherche de nouveaux composés organoleptiques répondant à la fois à des exigences réglementaires croissantes mais également en vue de remplacer les composés identifiés par le législateur comme indésirables voire inacceptables. Aussi, des contraintes de coût sont de plus en plus importantes.

Parmi les molécules organoleptiques, les composés dotés de notes fruitées, pêche et/ou fruits exotiques sont peu nombreux. On compte parmi les composés les plus utilisés la gamma-undécalactone, le Nectaryl® (Givaudan) ou l'Apritone® (Bedoukian) :

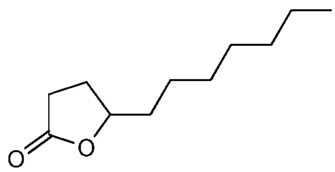

**Gamma-undecalactone**

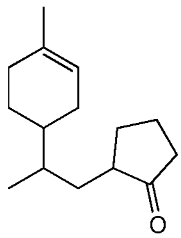

**Nectaryl®**

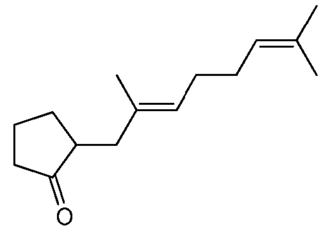

**Apritone®**

Cependant, les composés de l'art antérieur et notamment ceux ci-dessus présentent des notes fruitées et/ou fruits exotiques toujours accompagnées d'un côté gras, lactonique, crémeux, qui confère un aspect non naturel à l'arôme ou au parfum, ce qui n'est pas avantageux.

Aussi, afin de faire face aux besoins constants de l'industrie des parfums et arômes, et d'élargir la palette des parfumeurs et aromaticiens, la Demanderesse a identifié de nouveaux composés spirooxathiolanones présentant une note originale fruitée, pêche et/ou fruits exotiques, qui ont l'avantage de conférer un aspect naturel aux compositions. Ces composés présentent des notes suffisamment puissantes pour permettre une utilisation à une teneur finale très faible dans les compositions olfactives ou gustatives prêtes à emploi.

Ces composés spirooxathiolanones répondent à la formule (I) suivante :

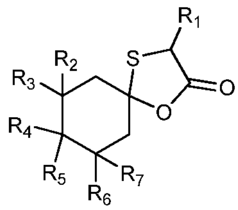

dans laquelle :

- $R_1$, $R_2$, $R_3$, $R_4$, $R_6$ et $R_7$, représentent indépendamment un atome d'hydrogène ou un groupe méthyle ;
- $R_5$ représente un atome d'hydrogène ou un groupe alkyle en C1-C5 linéaire saturé ;
- $R_4$ et $R_5$ pouvant former ensemble un groupe cyclopentyle ;
- le nombre total d'atomes de carbone étant strictement supérieur à 9.

La présente invention se rapporte également à une composition comprenant au moins un composé de formule générale (I).

En outre, un troisième objet de la présente invention se rapporte à un procédé de préparation d'un composé de formule générale (I), ledit procédé étant simple, avantageux en terme de rendement, comportant une seule étape et donc peu onéreux.

Enfin, un dernier objet de la présente invention concerne l'utilisation d'au moins un composé de formule générale

(I) pour conférer, modifier ou renforcer les propriétés organoleptiques d'une substance, d'une composition ou d'un article.

A la connaissance de la Demanderesse, aucun des composés répondant à la formule générale (I) n'a été identifié auparavant.

Des composés spirolactones utilisés en parfumerie ont été identifiés dans l'art antérieur, comme par exemple dans le brevet US 4519944 qui divulgue des composés de formule suivante :

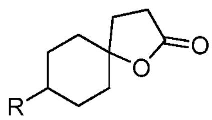

.

Ces composés sont non seulement différents structurellement des composés de la présente invention, mais ils présentent (pour les composés préférés) des notes boisée, laiteuse, lactonique, poudrée, notes qui sont donc bien différentes des composés décrits dans la présente invention.

Par ailleurs, des publications scientifiques divulguent certains composés spirooxathiolanones mais sans identifier leurs propriétés organoleptiques. Citons par exemple la 7-méthyl-1-oxa-4-thiaspiro[4.5]décan-2-one et la 3-méthyl-1-oxa-4-thiaspiro[4.5]décan-2-one (Tetrahedron, 1970, 26(19), 4641-4648) ainsi que la 8-tert-butyl-1-oxa-4-thiaspiro[4.5]décan-2-one (Synth. Commun., 2003, 33(11), 1951-1961).

Enfin, la demande de brevet JP 2001039972 (Hasegawa) décrit des oxathiolanones possédant une odeur de viande, de noisettes, culinaire, de formule générale :

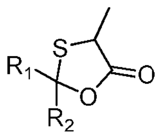

,

donc bien loin des notes fruitées, pêche et/ou exotique des composés décrits dans la présente invention.

Les composés spirooxathiolanes de la présente invention possèdent d'une part, une structure chimique bien distincte et nouvelle de celle des composés de l'art antérieur et, d'autre part, des notes fruitées, pêche, et/ou fruits exotiques octroyant un aspect naturel aux compositions et par rapport aux composés de référence. Par ailleurs, les composés selon l'invention ont des seuils de détection de l'odeur très faibles, notamment en comparaison avec le composé de référence qu'est la gamma-undécalactone. Toujours en comparaison avec la gamma-undécalactone, les composés spirooxathiolanes de la présente invention présentent une « valeur d'odeur » (ou « Odor Value », obtenue par le rapport de la volatilité/seuil de détection de l'odeur) beaucoup plus importante que celle de la gamma-undécalactone, ce qui démontre ainsi la puissance desdits composés par rapport à celle de la gamma-undécalactone.

Ainsi, la présente invention se rapporte à des composés spirooxathiolanones de formule générale (I) suivante :

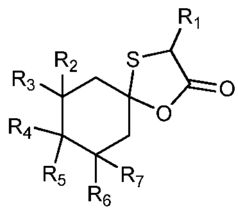

dans laquelle :

- $R_1$, $R_2$, $R_3$, $R_4$, $R_6$ et $R_7$, représentent indépendamment un atome d'hydrogène ou un groupe méthyle ;
- $R_5$ représente un atome d'hydrogène ou un groupe alkyle en C1-C5 linéaire saturé ;
- $R_4$ et $R_5$ pouvant former ensemble un groupe cyclopentyle ;
- le nombre total d'atomes de carbone étant strictement supérieur à 9.

Au sens de la présente invention, le terme « alkyle en $C_1$-$C_5$ » désigne tout radical monovalent dérivé d'une chaîne carbonée saturée, linéaire, contenant 1 ou 5 atomes de carbone, c'est à dire un groupement méthyle, éthyle, propyle, butyle et pentyle.

Selon un premier mode de réalisation, $R_5$ représente un groupe alkyle en $C_1$-$C_5$ linéaire saturé. Préférentiel-

lement, $R_5$ représente un groupe éthyle ou propyle.

Selon un autre mode de réalisation préféré, $R_1$, $R_2$, $R_3$, $R_4$, $R_6$ et $R_7$, représentent un atome d'hydrogène.

Plus particulièrement, le nombre total d'atomes de carbone est de 10 ou 11.

Selon un autre mode de réalisation préféré, le nombre total d'atomes de carbone est de 12.

Dans un mode de réalisation préféré, le composé selon la présente invention est choisi parmi la 7,7-diméthyl-1-oxa-4-thiaspiro[4.5]décan-2-one, la 8-éthyl-1-oxa-4-thiaspiro[4.5]décan-2-one, la 8-éthyl-3-méthyl-1-oxa-4-thiaspiro[4.5]décan-2-one, la 8,8-diméthyl-1-oxa-4-thiaspiro[4.5]décan-2-one, la 8-propyl-1-oxa-4-thiaspiro[4.5]décan-2-one, la 3-méthyl-8-propyl-1-oxa-4-thiaspiro[4.5]décan-2-one, l'oxathiolanone de la spiro[4.5]décan-8-one, la 7,7,9-triméthyl-1-oxa-4-thiaspiro[4.5]décan-2-one et la 8-pentyl-1-oxa-4-thiaspiro[4.5]décan-2-one.

La présence de centres d'asymétrie dans la structure des composés de formule (I) selon l'invention provoque l'existence, pour chacun d'entre eux, de plusieurs formes énantiomériques et/ou diastéréomériques. L'invention couvre également les composés représentés par la formule générale (I) sous forme de mélanges d'énantiomères et/ou de diastéréomères, en proportions variables, en particulier les mélanges racémiques. L'invention comprend également les composés de formule (I) sous forme d'un seul énantiomère et/ou diastéréomère. Des mélanges d'énantiomères/diastéréomères ou des formes pures peuvent être obtenus par synthèse à partir de produits de départ optiquement enrichis ou optiquement purs, ou au moyen de méthodes de séparation par cristallisation ou chromatographie.

Un second objet de la présente invention se rapporte à une composition comprenant au moins un composé de formule générale (I)

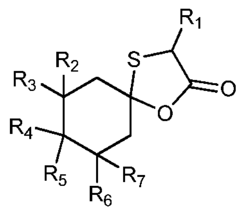

dans laquelle :

- $R_1$, $R_2$, $R_3$, $R_4$, $R_6$ et $R_7$, représentent indépendamment un atome d'hydrogène ou un groupe méthyle ;
- $R_5$ représente un atome d'hydrogène ou un groupe alkyle en $C_1$-$C_5$ linéaire saturé ;
- $R_4$ et $R_5$ pouvant former ensemble un groupe cyclopentyle ;
- le nombre total d'atomes de carbone étant strictement supérieur à 9.

Préférentiellement, une composition selon la présente invention comprend au moins un composé choisi parmi la 7,7-diméthyl-1-oxa-4-thiaspiro[4.5]décan-2-one, la 8-éthyl-1-oxa-4-thiaspiro[4.5]décan-2-one, la 8-éthyl-3-méthyl-1-oxa-4-thiaspiro[4.5]décan-2-one, la 8,8-diméthyl-1-oxa-4-thiaspiro[4.5]décan-2-one, la 8-propyl-1-oxa-4-thiaspiro[4.5]décan-2-one, la 3-méthyl-8-propyl-1-oxa-4-thiaspiro[4.5]décan-2-one, l'oxathiolanone de la spiro[4.5]décan-8-one, la 7,7,9-triméthyl-1-oxa-4-thiaspiro[4.5]décan-2-one et la 8-pentyl-1-oxa-4-thiaspiro[4.5]décan-2-one.

La quantité efficace des composés de l'invention à incorporer dans ces compositions est fonction de la nature desdites compositions, de l'effet odorant ou aromatisant souhaité et de la nature des autres composés odorants ou aromatisants éventuellement présents. Elle est aisément déterminée par l'homme du métier, et peut varier dans une plage très étendue, de 0,000001 à 50%, en particulier 0,000005 à 20%. Les pourcentages précédents sont exprimés en poids total de la composition.

Dans un premier mode de réalisation particulier, la composition selon l'invention est une composition de parfum comprenant au moins un composé de formule générale (I) et au moins une autre substance odorante. Les autres substances odorantes pouvant être utilisées en combinaison avec les composés de la présente invention peuvent être des produits naturels tels que des extraits, des huiles essentielles, des absolues, des résinoïdes, des résines, des concrètes etc., mais aussi des produits de synthèse tels que des hydrocarbures, alcools, aldéhydes, cétones, éthers, acides, esters, acétals, nitriles etc., notamment des composés saturés ou insaturés, aliphatiques, hétérocycliques ou carbonecycliques. De telles substances odorantes sont mentionnées par exemple, dans S. Arctander, « Perfume and Flavor Chemicals » (Montclair, N.J., 1969), ou encore dans « Common Fragrance and Flavor Materials », Wiley-VCH, Weinheim, 2006. Enfin, plusieurs composés de la présente invention peuvent également être utilisés en combinaison dans une même composition.

De par l'odeur plaisante qu'ils dégagent, les composés de l'invention trouvent nombre d'applications en parfumerie. Le terme "parfumerie" est ici utilisé dans son sens général ; il désigne non seulement la parfumerie traditionnelle (alcoolique ou non), mais également les autres domaines dans lesquels l'odeur des produits est importante. Il peut être ainsi fait référence à des compositions de parfumerie au sens habituel et traditionnel (telles que bases et concentrés

parfumant, parfums, eaux de Cologne, eaux de toilette, désodorisants d'intérieur, parfums d'ambiance, bougie parfumées et produits similaires), à des compositions topiques notamment cosmétiques (telles que crèmes pour le visage et/ou le corps, poudres de talc, huiles pour cheveux, shampooings, lotions capillaires, sels et huiles de bain, gels de douche et/ou de bain, savons de toilette, antiperspirants et déodorants corporels, lotions et crèmes de rasage, savons, dentifrices, bains de bouche, pommades, et produits similaires), ainsi qu'à des produits d'entretien, notamment ménagers (tels que détergents, lessives, assouplissants, désodorisants d'intérieur, parfums d'ambiance et produits similaires).

L'invention s'étend ainsi à une composition de parfum comprenant au moins un composé de l'invention. Il peut notamment s'agir d'une composition de la parfumerie traditionnelle, d'une composition cosmétique, de produit d'entretien, ou encore d'une "composition dite intermédiaire", destinée à être utilisée pour la préparation de compositions ou de produits finis (notamment parfums, produits cosmétiques, produits d'entretien).

Une telle composition parfumée est généralement préparée à partir d'un produit de base, dans lequel le ou les composés de l'invention se trouvent être incorporés. Le produit de base sera aisément déterminé par l'homme du métier en fonction de la composition envisagée et donc de l'utilisation envisagée. La composition de ces produits de base et la nature de leurs composants habituels, tels que solvant(s) et/ou adjuvant(s), sont bien connues de l'homme du métier.

Les composés entrant dans ces compositions parfumées, notamment les composés de l'invention, peuvent être incorporés dans ou sur un matériau support inerte. Les matériaux supports pouvant être employés sont nombreux et variés, par exemple des solvants polaires, des huiles, des graisses, des solides finement divisés, des cyclodextrines, des maltodextrines, des gommes, des résines et n'importe quel autre matériau support connu pour de telles compositions (par exemple, savons, bougies, pommades, textiles, lingettes, gels parfumés...).

Selon un autre mode de réalisation particulier, la composition selon l'invention est une composition aromatique comprenant au moins un composé de formule (I) et au moins une autre substance aromatique.

Plus particulièrement, la composition aromatique est un produit ingérable, qui se réfère à une "denrée alimentaire", un "composition comestible" et/ou un "produit alimentaire". La composition aromatique peut également être destinée à être utilisée dans le tabac. Ledit produit ingérable se rapporte préférentiellement, sans s'y limiter, à des produits destinés à l'alimentation humaine, destinés aux aliments pour animaux (animaux de compagnie) ou encore à des compositions pharmaceutiques. Des exemples de produits destinés à l'alimentation humaine peuvent inclure, sans s'y limiter, des collations, des confiseries, des matières végétales et des repas qui peuvent ou non fournir des nutriments essentiels. Les matières végétales comprennent le cacao, les fèves de cacao, le café, les grains de café et les feuilles de thé ou de la poudre. Des exemples non limitatifs de produits alimentaires comprennent vinaigrettes, sauces, sauces, marinades, bâtonnets, barres nutritionnelles, pâtisseries, pains, caramel, céréales cuites, produits de viande, produits de volaille, viande, volaille, volaille, poisson, sources de protéines marines, haricots, pâtes, produits de confiserie, collations salées, produits laitiers, fromages, yaourts, beurre, margarine, céréales prêtes à manger, condiments et sauces et boissons. En particulier, le terme "boisson" comprend des mélanges et des concentrés, y compris, mais sans limitation, des boissons alcoolisées et non alcoolisées prêtes à boire et des boissons en poudre sèches. Des exemples non limitatifs de boissons comprennent les boissons gazeuses, les boissons brassées, les produits laitiers, le yogourt à boire, le lait, les agents blanchissants pour le café, les boissons nutritionnelles. Des exemples non limitatifs d'aliments destinés aux animaux peuvent inclure: aliments pour animaux de compagnie, plus particulièrement chiens et chats aliments pour rongeurs, aliments pour bétail, aliments pour bovins, aliments pour chevaux et similaires.

Un troisième objet de la présente invention se rapporte à un procédé de préparation des composés de formule (I) tel que définis précédemment.

Ledit procédé est avantageux en ce sens qu'il est réalisé en une seule étape et permet l'utilisation de matières premières disponibles en grande quantité. Le rendement dudit procédé est également avantageux en ce qu'il est très élevé (près de 80%).

Les composés de la présente invention sont obtenus par une réaction de cyclisation entre une cycloalcanone de formule (II) et un thiolacide de formule (III) en présence d'un acide :

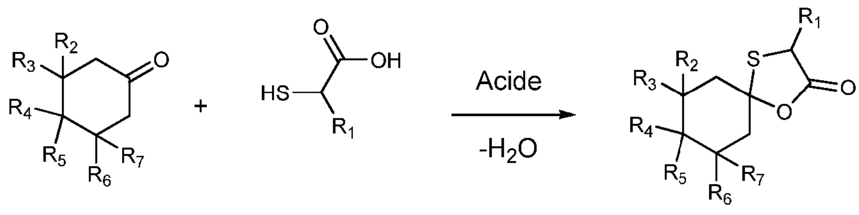


(II) (III)

dans laquelle :

- $R_1$, $R_2$, $R_3$, $R_4$, $R_6$ et $R_7$, représentent indépendamment un atome d'hydrogène ou un groupe méthyle ;
- $R_5$ représente un atome d'hydrogène ou un groupe alkyle en $C_1$-$C_5$ linéaire saturé ;
- $R_4$ et $R_5$ pouvant former ensemble un groupe cyclopentyle ;
- le nombre total d'atomes de carbone étant strictement supérieur à 9.

Dans un premier mode de réalisation, le thiolacide est l'acide thioglycolique.

Dans un second mode de réalisation, le thiolacide est l'acide thiolactique.

Préférentiellement, l'acide utilisé est l'acide *para*-toluène sulfonique. Encore plus préférentiellement, entre 1 à 5 % molaire d'acide para-toluène sulfonique sont utilisés par rapport aux réactifs. La réaction est notamment conduite à reflux à environ 70°C dans du cyclohexane.

Enfin, l'invention a pour dernier objet l'utilisation d'au moins un composé de formule (I) selon l'invention sous forme d'un stéréoisomère ou d'un mélange de stéréoisomères, ou d'un mélange racémique pour conférer, modifier ou renforcer les propriétés organoleptiques d'une substance, d'une composition ou d'un article.

On entend par «propriétés organoleptiques» toute propriété susceptible de modifier, améliorer ou renforcer la perception organoleptique d'une substance, d'une composition, d'un article par un utilisateur.

Dans un premier mode de réalisation, au moins un composé de formule (I) est utilisé en tant qu'agent fragrant, seul ou en combinaison avec au moins une autre substance odorante, et/ou au moins un solvant, et/ou au moins un adjuvant. Le ou les agents odorants supplémentaires, solvants et adjuvants sont connus de l'homme du métier qui sera à même de choisir le ou les plus appropriés en fonction de l'effet recherché.

Le terme «fragrant», est utilisé ici pour désigner tout composé organoleptique stimulant de façon plaisante l'odorat.

Les composés selon l'invention peuvent particulièrement être utilisés en tant qu'agent de masquage ou comme agent de neutralisation d'odeur. Par le terme «agent de masquage» ou «agent de neutralisation d'odeur» on entend réduire ou éliminer la perception d'une mauvaise odeur générée par une ou plusieurs molécules entrant dans la composition d'un produit.

Dans un second mode de réalisation, au moins un composé de formule générale (I) est utilisé en tant que composé aromatique, seul ou en combinaison avec au moins une autre substance aromatique et/ou au moins un solvant, et/ou au moins un adjuvant.

Le ou les agents aromatisants supplémentaires, solvants et adjuvants sont connus de l'homme du métier qui sera à même de choisir le ou les plus appropriés en fonction de l'effet recherché. Les solvants utilisés permettent non seulement un dosage exact du composé selon l'invention pour les aliments et les boissons, mais facilite également une distribution uniforme du composé selon l'invention dans les aliments et les boissons. Les solvants appropriés peuvent être des solvants hydrophiles comme l'eau, le propylène glycol, le glycérol, l'éthanol et la triacétine ou des solvants hydrophobes tels que les huiles végétales, par exemple huile de palme, huile de soja, huile de colza, huile de tournesol, huile d'arachide, triglycérides à chaîne moyenne (MCT). Les triglycérides à chaîne moyenne sont des triglycérides à base d'acides gras aliphatiques comprenant de 6 à 12 atomes de carbone.

Par aromatique, on entend toute utilisation des composés de l'invention pour l'aromatisation de toute denrée alimentaire liquide ou solide, humaine ou animale notamment des boissons, des produits laitiers, des crèmes glacées, mais également dans des applications d'aromatisation du tabac.

Particulièrement, les composés selon l'invention peuvent être utilisés seuls ou en combinaison avec des composés modulateurs du goût c'est-à-dire qui modifient le goût et les perceptions sensorielles. Dans tous les cas, la spécificité de tels composés modulateurs du goût est qu'ils ne présentent pas de goût et de propriétés aromatiques perceptibles (sans goût et sans arôme). De tels composés modifiant l'arôme peuvent être d'origine synthétique ou d'origine naturelle.

Les exemples suivants illustrent une manière particulière de préparer les composés de l'invention, ainsi que le profil olfactif/aromatique de chacun des composés exemplifiés. Ces exemples ne sont donnés que dans un but d'illustration et ne doivent pas être compris comme limitant la portée générale de l'invention.

### Exemple 1 : Préparation de la 7,7-diméthyl-1-oxa-4-thiaspiro[4.5]décan-2-one

La 3,3-diméthylcyclohexanone (dont la préparation est divulguée par exemple dans la demande WO 2010043522) est placée dans 1,1 équivalents d'acide thioglycolique et 4 volumes de cyclohexane. A température ambiante, on ajoute 0,05 équivalent d'acide para-toluène sulfonique. Le milieu réactionnel est porté à reflux tandis que l'eau formée est éliminée par distillation azéotropique. Lorsque la réaction est terminée, le milieu réactionnel est versé sur une solution aqueuse saturée en bicarbonate de sodium. La phase organique est lavée à l'eau jusqu'à pH neutre. Après séchage sur sulfate de magnésium, filtration et concentration, le produit brut est distillé sous pression réduite : sa température d'ébullition est de 89 °C sous 0,26 torr.

Description olfactive : fruité, effet pêche, framboise.

La 7,7-diméthyl-1-oxa-4-thiaspiro[4.5]décan-2-one ainsi obtenue présente les caractéristiques spectrales suivantes :

**$^1$H-RMN** (300 MHz, $CDCl_3$): δ (ppm) 3,73 (s, 1H), 3,72 (s, 1H), 2,10 - 1,90 (m, 2H), 1,85 - 1,58 (m, 4H), 1,46 - 1,29 (m, 1H), 1,34 - 1,16 (m, 1H), 1,04 (s, 3H), 0,97 (s, 3H).
**$^{13}$C-RMN** (75 MHz, $CDCl_3$): δ (ppm) 172,34, 92,16, 51,31, 39,82, 37,74, 32,23, 31,11, 28,37, 19,90.
**SM** [$EI^+$] (*m/z*) (%): 200 (M+, 9), 127(100), 109(55), 83(25), 69(34), 56(10), 55(35), 46(12), 43(26), 41(25), 39(10).
**IR** (pur, $cm^{-1}$): 2946m, 1765s, 1455w, 1215m, 1144m, 1060m, 1025m, 993m, 954m, 914w, 811w, 797w, 607w.

**Exemple 2 : Préparation de la 8-éthyl-1-oxa-4-thiaspiro[4.5]décan-2-one**

La 8-éthyl-1-oxa-4-thiaspiro[4.5]décan-2-one est préparée selon le protocole décrit en exemple 1 en utilisant la 4-éthylcyclohexanone à la place de la 3,3-diméthylcyclohexanone. Le produit brut, obtenu sous la forme de deux diastéréoisomères en proportions 54 : 46, est distillé sous pression réduite : sa température d'ébullition est de 98 °C sous 0,18 torr.

Description olfactive : fruits exotiques, mangue, goyave, papaye.

La 8-éthyl-1-oxa-4-thiaspiro[4.5]décan-2-one ainsi obtenue présente les caractéristiques spectrales suivantes :

Isomère majoritaire (54%) :

**$^1$H-RMN** (300 MHz, $CDCl_3$): δ (ppm) 3,62 (s, 2H), 2,21-2,06 (m, 1H), 2,05-1,88 (m, 2H), 1,80-1,60 (m, 3H), 1,40-1,23 (m, 1H), 1,26-1,04 (m, 4H), 0,81 (td, *J* = 7,2 Hz, 3H).
**$^{13}$C-RMN** (75 MHz, $CDCl_3$): δ (ppm) 172,24, 94,34, 38,70, 37,43, 31,69, 39,34, 11,57.
SM [$EI^+$] (*m/z*) (%): 200 (M+, 10), 127 (100), 109 (37), 67 (33), 55(41), 46(12), 43 (13), 41 (22).

Isomère minoritaire (46%) :

**$^1$H-RMN** (300 MHz, $CDCl_3$): δ (ppm) 3,69 (s, 2H), 2,21-2,06 (m, 1H), 2,05-1,88 (m, 2H), 1,80-1,60 (m, 3H), 1,40-1,23 (m, 1H), 1,26-1,04 (m, 4H), 0,81 (td, *J* = 7,2 Hz, 3H).
**$^{13}$C-RMN** (75 MHz, $CDCl_3$): δ (ppm) 172,26, 91,48, 39,49, 37,17, 32,25, 29,12, 28,69, 11,47.
**SM** [e/m (%)]: 200 (M+, 10), 129(10), 127 (100), 109 (35), 67 (33), 55(42), 46(12), 43 (14), 41 (23).
**IR** (pur, $cm^{-1}$): 2926m, 1767s, 1442w, 1197m, 1139m, 1041m, 966m, 915w, 892w, 854w, 796w.

**Exemple 3 : Préparation de la 8-éthyl-3-méthyl-1-oxa-4-thiaspiro[4.5]décan-2-one**

La 8-éthyl-3-méthyl-1-oxa-4-thiaspiro[4.5]décan-2-one est préparée selon le protocole décrit en exemple 1 en utilisant la 4-éthylcyclohexanone à la place de la 3,3-diméthylcyclohexanone, l'acide thiolactique (1,3 équivalents) à la place de l'acide thioglycolique et du toluène à la place du cyclohexane. Le produit brut, obtenu sous forme de deux diastéréoisomères en proportions 42 : 58 est distillé sous pression réduite : sa température d'ébullition est de 88 °C sous 0,4 mbar.

Description olfactive : pêche, vert, feuille de tomates.

La 8-éthyl-3-méthyl-1-oxa-4-thiaspiro[4.5]décan-2-one ainsi obtenue présente les caractéristiques spectrales suivantes :

Isomère majoritaire (58%) :

**$^1$H-RMN** (300 MHz, $CDCl_3$): δ (ppm) 3,99 (q, *J* = 7,0 Hz, 1H), 2,28 - 2,09 (m, 1H), 2,09 - 1,61 (m, 5H), 1,57 (d, *J* = 7,0 Hz, 3H), 1,52 - 0,95 (m, 5H), 0,88 (t, *J* = 7,0 Hz, 3H).
**$^{13}$C-RMN** (75 MHz, $CDCl_3$): δ (ppm) 175,19, 91,63, 40,90, 39,61, 38,96, 37,52, 29,78, 29,15, 28,71, 18,40, 11,60.
**SM** [$EI^+$] (*m/z*) (%): 210 (M+, 0,3), 195 (100), 137 (24), 109 (34), 101 (998), 93 (10), 91 (14), 81 (11), 79 (15), 76 (15), 67 (16), 43 (64), 41 (16).

Isomère minoritaire (42%) :

**$^1$H-RMN** (300 MHz, $CDCl_3$): δ (ppm) 3,99 (q, *J* = 7,0 Hz, 1H), 2,28 - 2,09 (m, 1H), 2,09 - 1,61 (m, 5H), 1,58 (d, *J* = 7,0 Hz, 3H), 1,52 - 0,95 (m, 5H), 0,87 (t, *J* = 7,0 Hz, 3H).
**$^{13}$C-RMN** (75 MHz, $CDCl_3$): δ (ppm) 175,25, 88,74, 41,43, 40,27, 39,74, 37,28, 29,23, 29,06, 28,76, 18,54, 11,50.
**SM** [$EI^+$] (*m/z*) (%): 214 [$M^+$] (6), 127 (100), 109 (19), 67 (12), 60 (20), 55 (23), 41 (15).
**IR** (film, $cm^{-1}$): 1039m, 1209m, 1447s, 1761s, 2928m.

**Exemple 4 : Préparation de la 8,8-diméthyl-1-oxa-4-thiaspiro[4.5]décan-2-one**

La 8,8-diméthyl-1-oxa-4-thiaspiro[4.5]décan-2-one est préparée selon le protocole décrit en exemple 1 en utilisant la 4,4-diméthylcyclohexanone à la place de la 3,3-diméthylcyclohexanone. Le produit brut est distillé sous pression réduite : sa température d'ébullition est de 92 °C sous 0,39 torr.

Description olfactive : fruité, vert, fruits exotiques.

La 8,8-diméthyl-1-oxa-4-thiaspiro[4.5]décan-2-one ainsi obtenue présente les caractéristiques spectrales suivantes :

**$^1$H-RMN** (300 MHz, $CDCl_3$): δ (ppm) 3,71 (s, 2H), 2,19-2,01 (m, 2H), 1,98-1,80 (m, 2H), 1,63-1,30 (m, 4H), 0,94 (2s, 6H).
**$^{13}$C-RMN** (75 MHz, $CDCl_3$): δ (ppm) 172,35, 93,19, 36,05, 35,74, 31,95, 29,11.
**SM** [$EI^+$] (*m/z*) (%): 200 (M+, 12), 127(100), 109 (35), 71(15), 67 (16), 55(33), 46 (15), 43(24), 41 (24), 39(10).
**IR** (pur, $cm^{-1}$): 2950m, 1767s, 1444w, 1232m, 1215m, 1156m, 1045s, 1001m, 972m, 877m, 791w, 585w.

**Exemple** 5 : **Préparation de la 8-propyl-1-oxa-4-thiaspiro[4.5]décan-2-one**

La 8-propyl-1-oxa-4-thiaspiro[4.5]décan-2-one est préparée selon le protocole décrit en exemple 1 en utilisant la 4-propylcyclohexanone à la place de la 3,3-diméthylcyclohexanone. Le produit brut, obtenu sous la forme de deux diastéréoisomères en proportions 46 : 54, est distillé sous pression réduite : sa température d'ébullition est de 106-110 °C sous 0,2 torr.

Description olfactive : pêche, abricot, juteux, pulpe.

La 8-propyl-1-oxa-4-thiaspiro[4.5]décan-2-one ainsi obtenue présente les caractéristiques spectrales suivantes :

Isomère majoritaire (54%) :

**$^1$H-RMN** (300 MHz, $CDCl_3$): δ (ppm) 3,74 (s, 2H), 2,22-2,16 (m, 1H), 2,05-1,95 (m, 2H), 1,85-1,64 (m, 3H), 1,40-1,11 (m, 7H), 0,89-0,84 (t, *J* = 7,2 Hz, 3H).
**$^{13}$C-RMN** (75 MHz, $CDCl_3$): δ (ppm) 172,38, 94,50, 38,79, 38,27, 31,78, 29,73, 20,12, 14,27.
**SM** [$EI^+$] (*m/z*) (%): 214 (M+, 7), 142(10), 141(100), 81(43), 67(20), 55(28), 46(10), 43(10), 41(18).

Isomère minoritaire (46%) :

**$^1$H-RMN** (300 MHz, $CDCl_3$): δ (ppm) 3,68 (s, 2H), 2,22-2,16 (m, 1H), 2,05-1,95 (m, 2H), 1,85-1,64 (m, 3H), 1,40-1,11 (m, 7H), 0,89-0,84 (t, *J* = 7,2 Hz, 3H).
**$^{13}$C-RMN** (75 MHz, $CDCl_3$): δ (ppm) 172,38, 91,65, 39,58, 38,24, 32,24, 29,50, 20,00, 14,27.
**MS** [e/m (%)]: 214 (M+, 9), 141(100), 81(39), 67(19), 55(22), 41(15).
**IR** (pur, $cm^{-1}$): 2926m, 1768s, 1443w, 1223m, 1193m, 1137w, 1043m, 969m, 912w, 842w, 796w, 589w.

**Exemple 6 : Préparation de la 3-méthyl-8-propyl-1-oxa-4-thiaspiro[4.5]décan-2-one**

La 3-méthyl-8-propyl-1-oxa-4-thiaspiro[4.5]décan-2-one est préparée selon le protocole décrit en exemple 1 en utilisant la 4-propylcyclohexanone à la place de la 3,3-diméthylcyclohexanone, l'acide thiolactique (1,3 équivalents) à la place de l'acide thioglycolique et le toluène à la place du cyclohexane. Le produit brut, obtenu sous la forme de deux diastéréoisomères en proportions 44 : 56, est distillé sous pression réduite : sa température d'ébullition est de 95

°C sous 0,4 mbar.

Description olfactive : pêche, fruité, vert.

La 3-méthyl-8-propyl-1-oxa-4-thiaspiro[4.5]décan-2-one ainsi obtenue présente les caractéristiques spectrales suivantes :

Isomère majoritaire (56%) :

**$^1$H-RMN** (300 MHz, $CDCl_3$): δ (ppm) 3,98 (q, *J* = 7,0 Hz, 1H), 2,26 - 1,61 (m, 6H), 1,56 (d, *J* = 7,0 Hz, 3H), 1,51 - 0,95 (m, 7H), 0,87 (t, *J* = 7,0 Hz, 3H).
**$^{13}$C-RMN** (75 MHz, $CDCl_3$): δ (ppm) 175,12, 91,56, 41,39, 40,86, 39,60, 38,95, 38,25, 35,48, 30,12, 20,11, 18,38, 14,26.
**SM** [$EI^+$] (*m/z*) (%): 228 [$M^+$] (3), 142 (10), 141 (100), 81 (20), 67 (10)

Isomère minoritaire (44%) :

**$^1$H-RMN** (300 MHz, $CDCl_3$): δ (ppm) 4,06 (q, *J* = 7,0 Hz, 1H), 2,26 - 1,61 (m, 6H), 1,57 (d, *J* = 7,0 Hz, 3H), 1,51 - 0,95 (m, 7H), 0,86 (t, *J* = 7,0 Hz, 3H).
**$^{13}$C-RMN** (75 MHz, $CDCl_3$): δ (ppm) 175,18, 88,68, 41,39, 40,24, 39,73, 38,30, 35,22, 29,56, 29,40, 19,99, 18,52, 14,26.
**SM** [$EI^+$] (*m/z*) (%): 228 [$M^+$] (3), 142 (10), 141 (100), 81 (20), 67 (10), 60 (13)
**IR** (film, $cm^{-1}$): 1036m, 1224m, 1443m, 1755s, 1926m.

**Exemple 7 : Préparation de l'oxathiolanone de la spiro[4.5]décan-8-one**

L'oxathiolanone de la spiro[4.5]décan-8-one est préparée selon le protocole décrit en exemple 1 en utilisant la spiro[4.5]décan-8-one à la place de la 3,3-diméthylcyclohexanone. Le produit brut est recristallisé dans le cyclohexane.

Description olfactive : pêche, velouté, vert, effet vanillé.

L'oxathiolanone de la spiro[4.5]décan-8-one ainsi obtenue présente les caractéristiques spectrales suivantes :

**$^1$H-RMN** (300 MHz, $CDCl_3$): (ppm) 3,74 (s, 2H), 2,16-2,07 (m, 2H), 1,97-1,88 (m, 2H), 1,69-1,59 (m, 6H), 1,54-1,43 (m, 6H).
**$^{13}$C-RMN** (75 MHz, $CDCl_3$): δ (ppm) 172,42, 93,38, 41,20, 37,02, 34,68, 32,00, 24,45, 24,39.
**SM** [$EI^+$] (*m/z*) (%): 226 (M+, 6), 154(11), 153(100), 135(10), 67(14), 55(15).
**IR** (pur, $cm^{-1}$): 2943m, 1771s, 1443m, 1267m, 1221s, 1209s, 1131m, 1041s, 978m, 933m, 900w, 838m, 794m, 608w.

**Exemple 8 : Préparation de la 7,7,9-triméthyl-1-oxa-4-thiaspiro[4.5]décan-2-one**

La 7,7,9-triméthyl-1-oxa-4-thiaspiro[4.5]décan-2-one est préparée selon le protocole décrit en exemple 1 en utilisant la 3,5,5-triméthylcyclohexanone à la place de la 3,3-diméthylcyclohexanone. Le produit brut, obtenu sous la forme de deux diastéréoisomères en proportions 74 : 26, est distillé sous pression réduite : sa température d'ébullition est de 87 °C sous 0,04 torr.

Description olfactive : pêche, boisé, camphré, vert.

La 7,7,9-triméthyl-1-oxa-4-thiaspiro[4.5]décan-2-one ainsi obtenue présente les caractéristiques spectrales suivantes :

Isomère majoritaire (74%) :

**$^1$H-RMN** (300 MHz, $CDCl_3$): δ (ppm) 3,73 (s, 2H), 2,19-2,06 (m, 1H), 2,04-1,84 (m, 2H), 1,57-1,25 (m, 3H), 1,06 (s, 3H), 0,97-0,80 (m, 7H).
**$^{13}$C-RMN** (75 MHz, $CDCl_3$): δ (ppm) 172,53, 91,83, 51,48, 48,37, 46,93, 33,48, 32,74, 32,18, 26,67, 25,98, 21,89.
**SM** [$EI^+$] (*m/z*) (%): 214 (M+, 7), 142(10), 141(100), 123(14), 83(85), 69(15), 55(25), 46(10), 43(11), 41(22).

Isomère minoritaire (26%) :

**$^1$H-RMN** (300 MHz, $CDCl_3$): δ (ppm) 3,65 (s, 2H), 2,19-2,06 (m, 1H), 2,04-1,84 (m, 2H), 1,57-1,25 (m, 3H), 1,04 (s, 3H), 0,97-0,80 (m, 7H).
**$^{13}$C-RMN** (75 MHz, $CDCl_3$): δ (ppm) 171,99, 93,57, 49,67, 47,62, 47,15, 33,56, 32,46, 32,29, 26,59, 26,38, 21,64.
**SM** [$EI^+$] (*m/z*) (%): 214 (M+, 5), 142(11), 141(100), 123(16), 83(88), 69(16), 55(25), 46(10), 43(13), 41(24), 39(10).
**IR** (pur, $cm^{-1}$): 2951m, 1766s, 1456w, 1210m, 1167m, 1139w, 1022m, 1005m, 958m, 896w, 859w, 798w, 612w.

**Exemple 9 : Préparation de la 8-pentyl-1-oxa-4-thiaspiro[4.5]décan-2-one**

La 8-pentyl-1-oxa-4-thiaspiro[4.5]décan-2-one est préparée selon le protocole décrit en exemple 1 en utilisant la 4-pentylcyclohexanone à la place de la 3,3-diméthylcyclohexanone. Le produit brut, obtenu sous la forme de deux diastéréoisomères en proportions 45 : 55, est distillé sous pression réduite : sa température d'ébullition est de 135 °C sous 0,2 torr.

Description olfactive : pêche, fruité, herbacé.

La 8-pentyl-1-oxa-4-thiaspiro[4.5]décan-2-one ainsi obtenue présente les caractéristiques spectrales suivantes :

Isomère majoritaire (55%) :

**$^1$H-RMN** (300 MHz, $CDCl_3$): δ (ppm) 3,67 (s, 2H), 2,21-2,14 (m, 1H), 2,06-1,90 (m, 2H), 1,84-1,64 (m, 3H), 1,40-1,11 (m, 11H), 0,88-0,83 (t, *J* = 7,2 Hz, 3H).
**$^{13}$C-RMN** (75 MHz, $CDCl_3$): δ (ppm) 172,32, 94,45, 38,79, 35,94, 35,76, 32,02, 31,99, 29,76, 22,60 14,06.
**SM** [$EI^+$] (*m/z*) (%): 242 (M+, 4), 170(12), 169(100), 95(24), 81(24), 67(12), 55(24), 43(12), 41(23).

Isomère minoritaire (45%) :

**$^1$H-RMN** (300 MHz, $CDCl_3$): δ (ppm) 3,73 (s, 2H), 2,21-2,14 (m, 1H), 2,06-1,90 (m, 2H), 1,84-1,64 (m, 3H), 1,40-1,11 (m, 11H), 0,88-0,83 (t, *J* = 7,2 Hz, 3H).
**$^{13}$C-RMN** (75 MHz, $CDCl_3$): δ (ppm) 172,32, 91,61, 39,58, 35,97, 35,49, 32,31, 31,75, 29,53, 26,58, 22,62, 14,27.
**SM** [e/m (%)]: 242 (M+, 4), 170(12), 169(100), 95(21), 81(21), 67(12), 55(25), 43(13), 41(23).
**IR** (pur, $cm^{-1}$): 2922m, 2853m, 1769s, 1443w, 1209m, 1184m, 1133w, 1041m, 982m, 901w, 796w.

**Exemple 10 : Composition parfumante comprenant les dérivés obtenus dans les exemples 2, 5 ou 7 appliquée dans une base de shampoing (à hauteur de 0,6%)**

Dans un accord rose réalisé selon le tableau suivant (Accord A) sont ajoutés :
- la 8-propyl-1-oxa-4-thiaspiro[4.5]décan-2-one (composé 16025-37, Exemple 5, Accord B)
- la 8-éthyl-1-oxa-4-thiaspiro[4.5]décan-2-one (composé 16025-43, Exemple 2, Accord C)
- l'oxathiolanone de la spiro[4.5]décan-8-one (composé 16025-56, Exemple 7, Accord D)

| **Ingrédients** | **A** | **B** | **C** | **D** |
|---|---|---|---|---|
| CITRONELLOL | 300 | 300 | 300 | 300 |
| GERANIOL | 150 | 150 | 150 | 150 |
| PHENYLETHYL ALCOHOL | 150 | 150 | 150 | 150 |
| PHENOXYETHYL ISOBUTYRATE | 80 | 80 | 80 | 80 |
| DIPHENYL OXIDE | 80 | 80 | 80 | 80 |
| NEROL | 75 | 75 | 75 | 75 |
| ISOAMYL ACETATE 10% DPG | 25 | 25 | 25 | 25 |
| GERANIUM ESS | 20 | 20 | 20 | 20 |

(suite)

| Ingrédients | A | B | C | D |
|---|---|---|---|---|
| ROSE OXIDE | 20 | 20 | 20 | 20 |
| CITRAL | 15 | 15 | 15 | 15 |
| OXACYCLOHEXADECAN-2-ONE | 15 | 15 | 15 | 15 |
| MAGNOLAN™ | 7 | 7 | 7 | 7 |
| DAMASCENONE 10% DPG | 7 | 7 | 7 | 7 |
| FRUCTONE™ | 5 | 5 | 5 | 5 |
| RASPBERRY KETONE | 5 | 5 | 5 | 5 |
| METHYL PHENYLETHYL ETHER 10% DPG | 5 | 5 | 5 | 5 |
| OXANE 50%TEC | 3 | 3 | 3 | 3 |
| DIMETHYL SULFIDE | 3 | 3 | 3 | 3 |
| VANILLINE | 1 | 1 | 1 | 1 |
| DIPROPYLENE GLYCOL - DPG | 34 | 29 | 29 | 29 |
| Composé 16025-37 | - | 5 | - | - |
| Composé 16025-43 | - | - | 5 | - |
| Composé 16025-56 | - | - | - | 5 |
| | 1000 | 1000 | 1000 | 1000 |

L'ajout de 5 parts du composé 16025-37 à l'accord A booste l'effet rosé-fruité de l'accord de manière élégante, donnant un effet plus pétale, naturel, tandis que l'ajout du composé 16025-43 dans les mêmes proportions apporte un effet plus boisé et pêche, donnant une rose plus riche et opulente. L'ajout du composé 16026-56 apporte une facette plus « dure », par le vert. L'accord est moins rosé que dans les deux cas précédents, plus fruité, pamplemousse et toujours avec plus de puissance que sur l'accord A.

Dans tous les cas, l'ajout d'une molécule selon l'invention apporte de la puissance et une note verte qui se marie bien à l'accord général de la composition parfumante.

**Exemple 11** : **Composition parfumante comprenant les dérivés obtenus dans les exemples 2, 5 ou 7 appliquée dans une base d'adoucissant pour linge (à hauteur de 1%)**

Dans un accord fruité-gourmand réalisé selon le tableau suivant (Accord A) sont ajoutés :

- la 8-propyl-1-oxa-4-thiaspiro[4.5]décan-2-one (composé 16025-37, Exemple 5, Accord B)
- la 8-éthyl-1-oxa-4-thiaspiro[4.5]décan-2-one (composé 16025-43, Exemple 2, Accord C)
- l'oxathiolanone de la spiro[4.5]décan-8-one (composé 16025-56, Exemple 7, Accord D)

| Ingrédients | A | B | C | D |
|---|---|---|---|---|
| HEXYLCINNAMIC ALDEHYDE | 125 | 125 | 125 | 125 |
| METHYL DIHYDRO JASMONATE | 80 | 80 | 80 | 80 |
| ETHYL VANILLINE | 75 | 75 | 75 | 75 |
| ISO E | 45 | 45 | 45 | 45 |
| HEXYL SALICYLATE | 45 | 45 | 45 | 45 |
| 4 tBu CYCLOHEXYL ACETATE | 40 | 40 | 40 | 40 |
| HABANOLIDE | 40 | 40 | 40 | 40 |

(suite)

| Ingrédients | A | B | C | D |
|---|---|---|---|---|
| VANILLINE | 38 | 38 | 38 | 38 |
| VERDYL ACETATE | 35 | 35 | 35 | 35 |
| 2 tBu CYCLOHEXYL ACETATE 50% DPG | 30 | 30 | 30 | 30 |
| ROSAFIX | 25 | 25 | 25 | 25 |
| TETRAHYDROLINALOL | 25 | 25 | 25 | 25 |
| ANISIC ALDEHYDE | 25 | 25 | 25 | 25 |
| FLOROL™ | 25 | 25 | 25 | 25 |
| PHENOXYETHYL ISOBUTYRATE | 25 | 25 | 25 | 25 |
| ORANGE TERPENES | 25 | 25 | 25 | 25 |
| GERANIOL | 23 | 23 | 23 | 23 |
| GAMMA UNDECALACTONE | 23 | 23 | 23 | 23 |
| PHENYLETHYL ALCOHOL | 18 | 18 | 18 | 18 |
| ETHYL METHYL PHENYL GLYCIDATE | 17 | 17 | 17 | 17 |
| GAMMA NONALACTONE | 17 | 17 | 17 | 17 |
| ETHYL MALTOL | 14 | 14 | 14 | 14 |
| SILVIAL™ 10% DPG | 14 | 14 | 14 | 14 |
| ALPHA ISOMETHYL IONONE | 12 | 12 | 12 | 12 |
| GERANYLE ACETATE 10% DPG | 12 | 12 | 12 | 12 |
| LINALYLE ACETATE | 10 | 10 | 10 | 10 |
| 1-(2,3-DIMETHYL-BICYCLO[2.2.1]HEPT-2-YL)-ETHANONE 1% DPG | 9 | 9 | 9 | 9 |
| COUMARINE | 7 | 7 | 7 | 7 |
| CIS-3-HEXENYL SALICYLATE | 7 | 7 | 7 | 7 |
| BENZYL ACETATE | 6 | 6 | 6 | 6 |
| METHYL ANTHRANILATE | 6 | 6 | 6 | 6 |
| FRAMBINONE | 6 | 6 | 6 | 6 |
| DMBC BUTYRATE | 6 | 6 | 6 | 6 |
| ETHYL CINNAMATE | 6 | 6 | 6 | 6 |
| MADERAL™ | 5 | 5 | 5 | 5 |
| DAMASCENONE 10% DPG | 5 | 5 | 5 | 5 |
| LIFFAROME™ 10% DPG | 5 | 5 | 5 | 5 |
| ETHYL METHYLVALERATE 10% DPG | 4 | 4 | 4 | 4 |
| DELTA DAMASCONE | 4 | 4 | 4 | 4 |
| HELIOTROPINE | 4 | 4 | 4 | 4 |
| CYCLOGALBANATE™ 10% DPG | 4 | 4 | 4 | 4 |
| PATCHOULY EO | 2 | 2 | 2 | 2 |
| 6-[2,4,4-TRIMETHYL-CYCLOPENTYLIDENE]-HEXANAL 1% DPG | 2 | 2 | 2 | 2 |
| ORCANOX™ | 2 | 2 | 2 | 2 |
| Composé 16025-37 | - | 3 | - | - |

(suite)

| Ingrédients | A | B | C | D |
|---|---|---|---|---|
| Composé 16025-43 | - | - | 3 | - |
| Composé 16025-56 | - | - | - | 3 |
| DIPROPYLENE GLYCOL - DPG | 50 | 47 | 47 | 47 |
| | 1000 | 1000 | 1000 | 1000 |

L'ajout de 3 parts du composé 16025-37 à l'accord A, apporte beaucoup de puissance et donne une note plus verte fusante, tandis que l'ajout du composé 16025-43 arrondit encore plus la note, avec un effet gourmand, vanillé plus présent et puissant.

L'ajout du composé 16025-56 arrondit aussi l'accord, tout en tassant les notes fruitées à ce dosage-là.

**Exemple 12 : Composition aromatique comprenant le dérivé obtenu dans l'exemple 5 appliquée dans un yaourt (à hauteur de 0,08% soit 160ppb)**

| Ingrédients | A | B |
|---|---|---|
| ACETYL METHYL CARBINOL 50% PG | 2 | 2 |
| STRAWBERRY FURANONE 30% PG | 3 | 3 |
| ACIDE PROPIONIQUE | 12,5 | 12,5 |
| ACIDE BUTYRIQUE | 15 | 15 |
| ALCOOL BUTYLIQUE | 15,5 | 15,5 |
| GAMMA DECALACTONE | 17,5 | 17,5 |
| ACIDE C02 ACETIQUE | 20 | 20 |
| ETHYLE ACETATE | 20 | 20 |
| LINALOL | 25 | 25 |
| ACIDE C05 BUTYRIQUE METHYL 2 | 25 | 25 |
| ABRICOT B.P.L. | 48,5 | 48,5 |
| PROPYLENE GLYCOL | 796 | 794,5 |
| Composé 16025-37 10% PG | - | 2 |
| | 1000 | 1000 |

L'ajout du composé 16025-37 à 160 ppb dans le yaourt confère à l'arôme pêche un profil plus authentique, plus rond, nectar de pêche.

**Exemple 13** : **Composition aromatique comprenant le dérivé obtenu dans l'exemple 5 appliquée dans un yaourt (à hauteur de 0,02% soit 140ppb)**

| Ingrédients | A | B |
|---|---|---|
| ACIDE BUTYRIQUE | 15 | 15 |
| GAMMA DECALACTONE | 7 | 7 |
| ACIDE C02 ACETIQUE | 12 | 12 |
| LINALOL | 1,5 | 1,5 |
| ACIDE C05 BUTYRIQUE METHYL 2 | 6 | 6 |
| THIAZOLE ISOPROPYL METHYL 1% ALC | 1 | 1 |
| BUCHU ESSENCE DETERPENE 1% ALC | 1,4 | 1,4 |
| GERANYLE ACETATE | 1,5 | 1,5 |

(suite)

| Ingrédients | A | B |
|---|---|---|
| GAMMA HEXALACTONE S | 2,5 | 2,5 |
| HEXYLE ACETATE | 3 | 3 |
| BENZOIQUE ALDEHYDE | 3 | 3 |
| DELTA DECALACTONE | 3 | 3 |
| MALTOL | 3 | 3 |
| GAMMA DODECALACTONE S | 3,5 | 3,5 |
| HEXENOL CIS 3 | 4,5 | 4,5 |
| HEXENYLE CIS 3 ACETATE | 9 | 9 |
| LIMONENE | 9 | 9 |
| ISOAMYLE ACETATE | 15 | 15 |
| ALCOOL C02 ETHYLIQUE | 899,1 | 897,1 |
| Composé 16025-37 10%PG | | 7 |
| | 1000 | 1000 |

L'ajout du composé 16025-37 à hauteur de 140 ppb complexifie le profil abricot, apporte un côté très naturel, chair d'abricot, jus, très bon. Le profil de cet arôme abricot est très particulier et n'a pu être reproduit à l'aide d'autres composés de l'art antérieur.

**Exemple 14 : Tests d'olfactométrie**

Il est généralement admis que le rapport de la volatilité par le seuil de détection d'une odeur permet l'obtention d'une «valeur d'odeur » (ou « O*dor Value* »), sans unité, rendant compte de la puissance olfactive d'une molécule. Plus cette valeur est élevée, plus la molécule en question est puissante. Afin de calculer cette Odor Value, la volatilité ainsi que le seuil de détection doivent par conséquent être déterminés.

Dans la présente étude, deux molécules de l'invention sont testées afin de déterminer leur puissance (via leur Odor Value) par rapport à une molécule de référence de l'art antérieur, la gamma-undécalactone. Les deux molécules selon l'invention qui sont testées sont celle de l'exemple 2 (la 8-éthyl-1-oxa-4-thiaspiro[4.5]décan-2-one) et celle de l'exemple 5 (la 8-propyl-1-oxa-4-thiaspiro[4.5]décan-2-one).

Dans un premier temps, les valeurs de volatilité à 20°C des molécules de l'exemple 2 et de l'exemple 5 sont déterminées par ébulliométrie. Ces valeurs sont respectivement de 19,8 $\mu g.l^{-1}$ et de 7,8 $\mu g.l^{-1}$.

Dans un second temps, des études psychosensorielles ont été menées afin de déterminer le seuil de détection des deux molécules selon la présente invention et ainsi que celui de de la gamma-undécalactone.

La valeur du seuil de détection est obtenue à l'aide d'un olfactomètre dynamique construit selon les lignes directrices des normes ISO 13725 et ISO 13301. Elle correspond à la concentration gazeuse statistique critique nécessaire pour déclencher une réponse positive pour un ensemble de dix-huit panélistes minimum dont l'âge varie de 22 à 57 ans selon un modèle d'un choix forcé parmi deux. L'un de ces choix est la dilution contrôlée d'un flux d'air saturé en matière première, l'autre choix est un flux d'air neutre. La répétition aléatoire de ces choix pour un ensemble de cinq à huit concentrations gazeuses permet ainsi la détermination du seuil de détection après traitement statistique des données obtenues. Ainsi, le seuil de détection pour les molécules de l'exemple 2 et de l'exemple 5 ont été déterminés et sont respectivement de 0,016 $ng.l^{-1}$ et 0,075 $ng.l^{-1}$.

Ainsi, après ces mesures, il est possible de calculer l'Odor Value des molécules selon l'invention en comparaison à celle de la gamma-undécalactone.

$$\text{Odor Value} = \text{Valeur de volatilité} / \text{Seuil de détection}$$

L'Odor Value est égale à 1 222 223 pour la molécule selon l'exemple 2, et à 104 133 pour la molécule selon l'exemple 5. Or, pour la gamma-undécalactone l'Odor Value déterminée dans des conditions expérimentales identiques est de 5 473.

En conclusion, l'Odor Value des molécules selon l'invention (des exemples 2 et 5) est très nettement supérieur à celle de la gamma-undécalactone ce qui indique que lesdites molécules selon l'invention sont très nettement plus puissantes que la gamma-undécalactone.

En outre, la substantivité évaluée par les panélistes est égale à 2,59 pour la molécule selon l'exemple 2, et à 2,62 pour la molécule selon l'exemple 5.

## Revendications

1. Composé de formule générale (I) suivante :

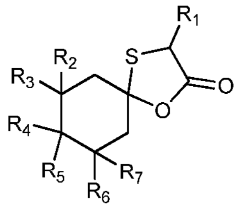

(I)

dans laquelle :

- $R_1$, $R_2$, $R_3$, $R_4$, $R_6$ et $R_7$, représentent indépendamment un atome d'hydrogène ou un groupe méthyle ;
- $R_5$ représente un atome d'hydrogène ou un groupe alkyle en $C_1$-$C_5$ linéaire saturé ;
- $R_4$ et $R_5$ pouvant former ensemble un groupe cyclopentyle ;
- le nombre total d'atomes de carbone étant strictement supérieur à 9.

2. Composé selon la revendication 1 **caractérisé en ce que** $R_5$ représente un groupe alkyle en $C_1$-$C_5$ linéaire saturé.

3. Composé selon la revendication 1 ou 2 **caractérisé en ce que** $R_1$, $R_2$, $R_3$, $R_4$, $R_6$ et $R_7$, représentent un atome d'hydrogène.

4. Composé selon l'une quelconque des revendications 1 à 3 **caractérisé en ce que** le nombre total d'atomes de carbone est de 10 ou 11.

5. Composé selon l'une quelconque des revendications précédentes **caractérisé en ce qu'**il est choisi parmi la 7,7-diméthyl-1-oxa-4-thiaspiro[4.5]décan-2-one, la 8-éthyl-1-oxa-4-thiaspiro[4.5]décan-2-one, la 8-éthyl-3-méthyl-1-oxa-4-thiaspiro[4.5]décan-2-one, la 8,8-diméthyl-1-oxa-4-thiaspiro[4.5]décan-2-one, la 8-propyl-1-oxa-4-thiaspiro[4.5]décan-2-one, la 3-méthyl-8-propyl-1-oxa-4-thiaspiro[4.5]décan-2-one, l'oxathiolanone de la spiro[4.5]décan-8-one, la 7,7,9-triméthyl-1-oxa-4-thiaspiro[4.5]décan-2-one et la 8-pentyl-1-oxa-4-thiaspiro[4.5]décan-2-one.

6. Composition comprenant au moins un composé de formule générale (I) suivante :

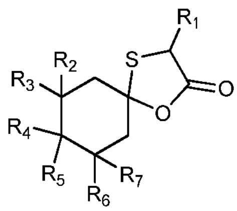

dans laquelle :

- $R_1$, $R_2$, $R_3$, $R_4$, $R_6$ et $R_7$, représentent indépendamment un atome d'hydrogène ou un groupe méthyle ;
- $R_5$ représente un atome d'hydrogène ou un groupe alkyle en C1-C5 linéaire saturé ;
- $R_4$ et $R_5$ pouvant former ensemble un groupe cyclopentyle ;
- le nombre total d'atomes de carbone étant strictement supérieur à 9.

7. Composition selon la revendication 6, ladite composition comprenant au moins un composé choisi parmi la 7,7-diméthyl-1-oxa-4-thiaspiro[4.5]décan-2-one, la 8-éthyl-1-oxa-4-thiaspiro[4.5]décan-2-one, la 8-éthyl-3-méthyl-1-oxa-4-thiaspiro[4.5]décan-2-one, la 8,8-diméthyl-1-oxa-4-thiaspiro[4.5]décan-2-one, la 8-propyl-1-oxa-4-thiaspiro[4.5]décan-2-one, la 3-méthyl-8-propyl-1-oxa-4-thiaspiro[4.5]décan-2-one, l'oxathiolanone de la spiro[4.5]décan-8-one, la 7,7,9-triméthyl-1-oxa-4-thiaspiro[4.5]décan-2-one et la 8-pentyl-1-oxa-4-thiaspiro[4.5]décan-2-one.

**8.** Composition selon la revendication 6 ou 7 **caractérisée en ce que** le composé de formule (I) est présent dans une concentration comprise entre 0,000001 à 50% en poids par rapport au poids total de la composition, plus particulièrement entre 0,000005 à 20%.

**9.** Composition selon l'une quelconque des revendications 6 à 8 **caractérisée en ce qu'**il s'agit d'une composition de parfum comprenant au moins un composé de formule (I) et au moins une autre substance odorante.

**10.** Composition selon l'une quelconque des revendications 6 à 8 **caractérisée en ce qu'**il s'agit d'une composition aromatique comprenant au moins un composé de formule (I) et au moins une autre substance aromatique.

**11.** Procédé de préparation d'un composé de formule (I) tel que décrit dans les revendications 1 à 5 par une réaction de cyclisation entre une cycloalcanone de formule (II) et un thiolacide de formule (III) en présence d'un acide

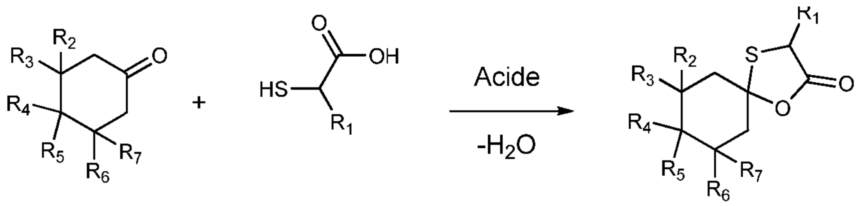


(II) (III)

dans lesquelles :

- $R_1$, $R_2$, $R_3$, $R_4$, $R_6$ et $R_7$, représentent indépendamment un atome d'hydrogène ou un groupe méthyle ;
- $R_5$ représente un atome d'hydrogène ou un groupe alkyle en $C_1$-$C_5$ linéaire saturé ;
- $R_4$ et $R_5$ pouvant former ensemble un groupe cyclopentyle ;
- le nombre total d'atomes de carbone étant strictement supérieur à 9.

**12.** Procédé selon la revendication 11 **caractérisé en ce que** le thiolacide est l'acide thioglycolique.

**13.** Procédé selon la revendication 11 **caractérisé en ce que** le thiolacide est l'acide thiolactique.

**14.** Procédé selon l'une quelconque des revendications 11 à 13 **caractérisé en ce que** l'acide utilisé est l'acide paratoluène sulfonique.

**15.** Utilisation d'au moins un composé de formule générale (I) tel que défini aux revendications 1 à 5 sous forme d'un stéréoisomère ou d'un mélange de stéréoisomères, ou d'un mélange racémique pour conférer, modifier ou renforcer les propriétés organoleptiques d'une substance, d'une composition ou d'un article.

**16.** Utilisation selon la revendication 15 d'au moins un composé de formule (I) en tant qu'agent fragrant, seul ou en combinaison avec au moins une autre substance odorante, et/ou au moins un solvant, et/ou au moins un adjuvant.

**17.** Utilisation selon la revendication 15 d'au moins un composé de formule générale (I) en tant que composé aromatique, seul ou en combinaison avec au moins une autre substance aromatique et/ou au moins un solvant, et/ou au moins un adjuvant.

**Patentansprüche**

**1.** Verbindung der folgenden allgemeinen Formel (I):

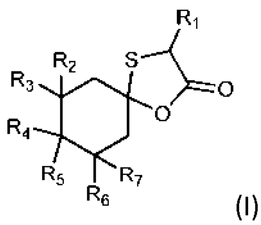

(I)

worin:

- $R_1$, $R_2$, $R_3$, $R_4$, $R_6$ und $R_7$ unabhängig voneinander ein Wasserstoffatom oder eine Methylgruppe darstellen;
- $R_5$ ein Wasserstoffatom oder eine gesättigte lineare $C_1$-$C_5$ -Alkylgruppe darstellt;
- $R_4$ und $R_5$ zusammen eine Cyclopentylgruppe bilden können;
- die Gesamtzahl der Kohlenstoffatome streng größer als 9 ist.

2. Verbindung nach Anspruch 1, **dadurch gekennzeichnet, dass** $R_5$ eine gesättigte lineare $C_1$-$C_5$-Alkylgruppe darstellt.

3. Verbindung nach Anspruch 1 oder 2, **dadurch gekennzeichnet, dass** $R_1$, $R_2$, $R_3$, $R_4$, $R_6$ und $R_7$ ein Wasserstoffatom darstellen.

4. Verbindung nach einem der Ansprüche 1 bis 3, **dadurch gekennzeichnet, dass** die Gesamtzahl der Kohlenstoffatome 10 oder 11 ist.

5. Verbindung nach einem der vorstehenden Ansprüche, **dadurch gekennzeichnet, dass** sie unter 7,7-Dimethyl-1-oxa-4-thiaspiro[4.5]decan-2-on, 8-Ethyl-1-oxa-4-thiaspiro[4.5]decan-2-on, 8-Ethyl-3-methyl-1-oxa-4-thiaspiro[4.5]decan-2-on, 8,8-Dimethyl-1-oxa-4-thiaspiro[4.5]decan-2-on, 8-Propyl-1-oxa-4-thiaspiro[4.5]decan-2-on, 3-Methyl-8-propyl-1-oxa-4-thiaspiro[4.5]decan-2-on, Oxathiolanon aus Spiro[4.5]decan-8-on, 7,7,9-Trimethyl-1-oxa-4-thiaspiro[4.5]decan-2-on und 8-Pentyl-1-oxa-4-thiaspiro[4.5]decan-2-on ausgewählt ist.

6. Zusammensetzung, die zumindest eine Verbindung der folgenden allgemeinen Formel (I) umfasst:

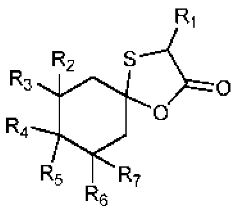

worin:

- $R_1$, $R_2$, $R_3$, $R_4$, $R_6$ und $R_7$ unabhängig voneinander ein Wasserstoffatom oder eine Methylgruppe darstellen;
- $R_5$ ein Wasserstoffatom oder eine gesättigte lineare $C_1$-$C_5$ -Alkylgruppe darstellt;
- $R_4$ und $R_5$ zusammen eine Cyclopentylgruppe bilden können;
- die Gesamtzahl der Kohlenstoffatome streng größer als 9 ist.

7. Zusammensetzung nach Anspruch 6, wobei besagte Verbindung zumindest eine Verbindung umfasst, die aus 7,7-Dimethyl-1-oxa-4-thiaspiro[4.5]decan-2-on, 8-Ethyl-1-oxa-4-thiaspiro[4.5]decan-2-on, 8-Ethyl-3-methyl-1-oxa-4-thiaspiro[4.5]decan-2-on, 8,8-Dimethyl-1-oxa-4-thiaspiro[4.5]decan-2-on, 8-Propyl-1-oxa-4-thiaspiro[4.5]decan-2-on, 3-Methyl-8-propyl-1-oxa-4-thiaspiro[4.5]decan-2-on, Oxathiolanon aus Spiro[4.5]decan-8-on, 7,7,9-Trimethyl-1-oxa-4-thiaspiro[4.5]decan-2-on und 8-Pentyl-1-oxa-4-thiaspiro[4.5]decan-2-on ausgewählt ist.

8. Zusammensetzung nach Anspruch 6 oder 7, **dadurch gekennzeichnet, dass** die Verbindung der Formel (I) in einer Konzentration von 0,000001 bis 50 Gew.-%, bezogen auf das Gesamtgewicht der Verbindung, insbesondere 0,000005 bis 20 %, vorliegt.

**9.** Zusammensetzung nach einem der Ansprüche 6 bis 8, **dadurch gekennzeichnet, dass** es sich um eine Parfümzusammensetzung handelt, die zumindest eine Verbindung der Formel (I) und zumindest einen weiteren Geruchstoff enthält.

**10.** Zusammensetzung nach einem der Ansprüche 6 bis 8, **dadurch gekennzeichnet, dass** es sich um eine aromatische Zusammensetzung handelt, die zumindest eine Verbindung der Formel (I) und zumindest eine weitere aromatische Substanz umfasst.

**11.** Verfahren zur Herstellung einer Verbindung der Formel (I), wie in den Ansprüchen 1 bis 5 beschrieben, durch eine Cyclisationsreaktion zwischen einem Cycloalkanon der Formel (II) und einer Thiosäure der Formel (III) in Gegenwart einer Säure

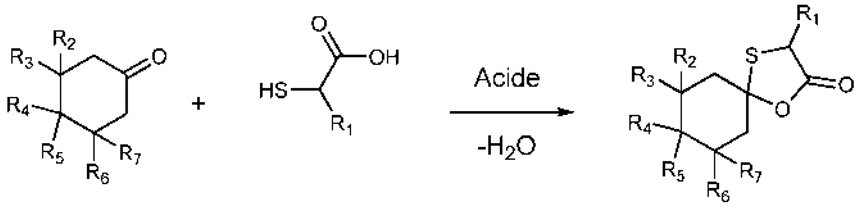


(II) (III)

worin:

- $R_1$, $R_2$, $R_3$, $R_4$, $R_6$ und $R_7$ unabhängig voneinander ein Wasserstoffatom oder eine Methylgruppe darstellen;
- $R_5$ ein Wasserstoffatom oder eine gesättigte lineare $C_1$-$C_5$ -Alkylgruppe darstellt;
- $R_4$ und $R_5$ zusammen eine Cyclopentylgruppe bilden können;
- die Gesamtzahl der Kohlenstoffatome streng größer als 9 ist.

**12.** Verfahren nach Anspruch 11, **dadurch gekennzeichnet, dass** die Thiosäure Thioglykolsäure ist.

**13.** Verfahren nach Anspruch 11, **dadurch gekennzeichnet, dass** die Thiosäure Thiomilchsäure ist.

**14.** Verfahren nach einem der Ansprüche 11 bis 13, **dadurch gekennzeichnet, dass** als Säure para-Toluolsulfonsäure verwendet wird.

**15.** Verwendung zumindest einer Verbindung der allgemeinen Formel (I), wie in den Ansprüchen 1 bis 5 definiert, in Form eines Stereoisomers oder eines Gemisches von Stereoisomeren oder eines racemischen Gemisches, um die organoleptischen Eigenschaften einer Substanz, einer Zusammensetzung oder eines Gegenstands zu verleihen, zu modifizieren oder zu verstärken.

**16.** Verwendung nach Anspruch 15 zumindest einer Verbindung der Formel (I) als Duftmittel, allein oder in Kombination mit zumindest einem weiteren Duftstoff und/oder zumindest einem Lösungsmittel und/oder zumindest einem Zusatzstoff.

**17.** Verwendung nach Anspruch 15 zumindest einer Verbindung der allgemeinen Formel (I) als aromatische Verbindung, allein oder in Kombination mit zumindest einem weiteren Aromastoff und/oder zumindest einem Lösungsmittel und/oder zumindest einem Zusatzstoff.

## Claims

**1.** A compound of the following general formula (I):

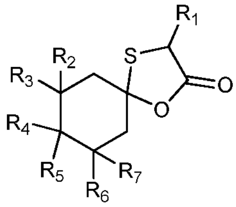

(I)

wherein:

- $R_1$, $R_2$, $R_3$, $R_4$, $R_6$ and $R_7$, independently represent a hydrogen atom or a methyl group;
- $R_5$ represents a hydrogen atom or a saturated linear $C_1$-$C_5$ alkyl group;
- $R_4$ and $R_5$ may together form a cyclopentyl group;
- the total number of carbon atoms being strictly greater than 9.

2. The compound according to claim 1, **characterised in that** $R_5$ represents a saturated linear $C_1$-$C_5$ alkyl group.

3. The compound according to claim 1 or 2 **characterised in that** $R_1$, $R_2$, $R_3$, $R_4$, $R_6$ and $R_7$, represent a hydrogen atom.

4. The compound according to any one of claims 1 to 3 **characterised in that** the total number of carbon atoms is 10 or 11.

5. The compound according to any one of the preceding claims, **characterised in that** it is selected from the 7,7-dimethyl-1-oxa-4-thiaspiro[4.5]decan-2-one, 8-ethyl-1-oxa-4-thiaspiro[4.5]decan-2-one, 8-ethyl-3-methyl-1-oxa-4-thiaspiro[4.5]decan-2-one, 8,8-dimethyl-1-oxa-4-thiaspiro[4.5]decan-2-one, 8-propyl-1-oxa-4-thiaspiro[4.5]decan-2-one, 3-methyl-8-propyl-1-oxa-4-thiaspiro[4.5]decan-2-one, oxathiolanone of the spiro[4.5]decan-8-one, 7,7,9-trimethyl-1-oxa-4-thiaspiro[4.5]decan-2-one and 8-pentyl-1-oxa-4-thiaspiro[4.5]decan-2-one.

6. A composition comprising at least one compound of the following general formula (I):

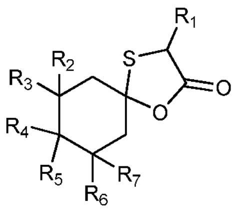

wherein:

- $R_1$, $R_2$, $R_3$, $R_4$, $R_6$ and $R_7$, independently represent a hydrogen atom or a methyl group;
- $R_5$ represents a hydrogen atom or a saturated linear $C_1$-$C_5$ alkyl group;
- $R_4$ and $R_5$ may together form a cyclopentyl group;
- the total number of carbon atoms being strictly greater than 9.

7. The composition according to claim 6, said composition comprising at least one compound selected from the 7,7-dimethyl-1-oxa-4-thiaspiro[4.5]decan-2-one, 8-ethyl-1-oxa-4-thiaspiro[4.5]decan-2-one, 8-ethyl-3-methyl-1-oxa-4-thiaspiro[4.5]decan-2-one, 8,8-dimethyl-1-oxa-4-thiaspiro[4.5]decan-2-one, 8-propyl-1-oxa-4-thiaspiro[4.5]decan-2-one, 3-methyl-8-propyl-1-oxa-4-thiaspiro[4.5]decan-2-one, oxathiolanone of the spiro[4.5]decan-8-one, 7,7,9-trimethyl-1-oxa-4-thiaspiro[4.5]decan-2-one and 8-pentyl-1-oxa-4-thiaspiro[4.5]decan-2-one.

8. The composition according to claim 6 or 7, **characterized in that** the compound of formula (I) is present in a concentration of between 0.000001 to 50% by weight with respect to the total weight of the composition, more particularly between 0.000005 to 20%.

9. The composition according to any one of claims 6 to 8 **characterized in that** it is a perfume composition comprising at least one compound of formula (I) and at least one other odorant.

**10.** The composition according to any one of claims 6 to 8 **characterised in that** it is an aromatic composition comprising at least one compound of formula (I) and at least one other aromatic substance.

**11.** A method for the preparation of a compound of formula (I) as described in claims 1 to 5 by a cyclization reaction between a cycloalkanone of formula (II) and a thiolacid of formula (III) in the presence of an acid

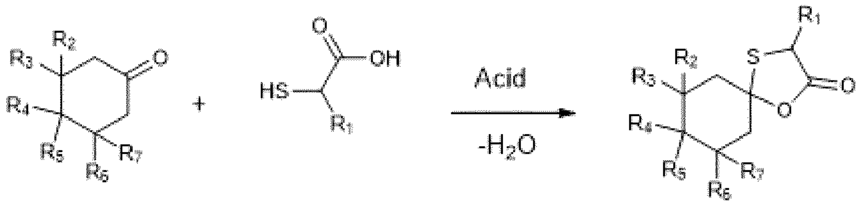


(II) (III)

wherein:

- $R_1$, $R_2$, $R_3$, $R_4$, $R_6$ and $R_7$, independently represent a hydrogen atom or a methyl group;
- $R_5$ represents a hydrogen atom or a saturated linear $C_1$-$C_5$ alkyl group;
- $R_4$ and $R_5$ may together form a cyclopentyl group;
- the total number of carbon atoms being strictly greater than 9.

**12.** The method according to claim 11, **characterised in that** the thiolacid is thioglycolic acid.

**13.** The method according to claim 11 **characterised in that** the thiolacid is thiolactic acid.

**14.** The method according to any one of claims 11 to 13 **characterised in that** the acid used is *para*-toluene sulphonic acid.

**15.** Use of at least one compound of general formula (I) as defined in claims 1 to 5 in the form of a stereoisomer or a mixture of stereoisomers, or a racemic mixture for conferring, modifying or enhancing the organoleptic properties of a substance, composition or article.

**16.** Use according to claim 15 of at least one compound of formula (I) as a fragrance agent, alone or in combination with at least one other odorant, and/or at least one solvent, and/or at least one adjuvant.

**17.** Use according to claim 15 of at least one compound of the general formula (I) as an aromatic compound, alone or in combination with at least one other aromatic substance and/or at least one solvent, and/or at least one adjuvant.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 4519944 A
- JP 2001039972 B, Hasegawa
- WO 2010043522 A

### Littérature non-brevet citée dans la description

- *Tetrahedron,* 1970, vol. 26 (19), 4641-4648
- *Synth. Commun.,* 2003, vol. 33 (11), 1951-1961
- **S. ARCTANDER.** Perfume and Flavor Chemicals. Montclair, 1969
- Common Fragrance and Flavor Materials. Wiley-VCH, 2006